(19) Europäisches Patentamt
European Patent Office
Office européen des brevets

(11) **EP 3 666 179 A1**

(12) **EUROPEAN PATENT APPLICATION**

(43) Date of publication:
**17.06.2020 Bulletin 2020/25**

(51) Int Cl.:
**A61B 5/01** (2006.01)       **G01K 1/20** (2006.01)
**G01K 13/00** (2006.01)

(21) Application number: **18211550.1**

(22) Date of filing: **11.12.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **BLOM, Antonius Hermanus Maria**
  **5656 AE Eindhoven (NL)**

• **CIUHU, Calina**
  **5656 AE Eindhoven (NL)**
• **ATALLAH, Louis Nicolas**
  **5656 AE Eindhoven (NL)**
• **BONGERS, Edwin Gerardus Johannus Maria**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **CORE BODY TEMPERATURE SENSOR SYSTEM BASED ON FLUX MEASUREMENT**

(57)     A core body temperature sensor system which has multiple pairs of temperature sensors across a first insulating layer. The sensed temperatures are monitored as well as the rate of change of sensed temperature at the temperature sensors. The core body temperature is obtained based on a first representative temperature for the first set of temperature sensors, a second representative temperature for the second set of temperature sensors and rate of change information.

FIG. 4

**Description**

FIELD OF THE INVENTION

This invention relates to a temperature sensor system for measuring a core body temperature based on a flux measurement. The invention relates in particular to a passive temperature sensor system.

BACKGROUND OF THE INVENTION

The core body temperature (CBT) is the operating temperature in deep structures of the body such as the liver, brain and heart. It is normally maintained within a narrow range for healthy subjects (36.5°C - 37.5°C) so that the essential homeostasis is not disturbed. However, this is not the case for several groups of patients both in and out of hospital. A change in CBT can be due to interventions (such as anesthesia during surgery), pathologies, infections and deteriorating conditions. For surgical patients, having intraoperative core hypothermia causes serious complications including surgical wound infections and myocardial complications. It also decreases drug metabolism, prolongs recovery, and provokes thermal discomfort. Therefore, clinicians ensure that patients are warmed enough, and their temperature is maintained in a comfortable range throughout the peri-operative journey.

Direct measurement of CBT involves using probes (such as esophageal or rectal probes) which are not practical for awake and ambulatory patients. Alternatively, CBT is estimated by using mercury or in-ear thermometers (infrared sensors). These measurements are not continuous and can suffer from inaccuracy.

Non-invasive measurement of core body temperature has been proposed via active or passive sensors.

A known design of active temperature sensor for measuring a core body temperature comprises a first temperature sensor, a thermal insulator, a second temperature sensor and a heating element.

When the temperature sensing device is positioned against the skin of the body the first temperature sensor measures the skin temperature. Assuming a core body temperature higher than the ambient temperature, there will be a decreasing temperature gradient in the sensing device in the direction away from the contact area between the temperature sensing device and the skin: the further a specific position inside the temperature sensing device is away from the contact area, the lower the temperature at the specific position will be. Especially, the thermal insulator creates a significant step in this gradient. Consequently, the second temperature sensor will measure a lower temperature than the first temperature sensor. As a result of the decreasing temperature gradient a heat flux will be present through the temperature sensor in the direction away from the contact area.

There is a temperature gradient in the body of the person or animal as well. The core of the body has a higher temperature than the skin so that a heat flux is present from the core of the body towards the skin as well as a heat flux through the temperature sensing device as the result of the temperature gradient in the device itself.

The measured temperature difference between the first temperature sensor and the second temperature sensor is a measure for the heat flux from the skin contact area toward the top of the temperature sensing device.

The measured temperature difference is then used to control the heating element. If the top of the sensor is heated, the temperature gradient inside the sensing device, created by the thermal insulation, partly disappears. As a consequence, the heat flux from the contact area between the temperature sensing device and the skin of the body towards the top of the sensing device reduces. The skin of the body becomes warmer and closer to the core body temperature. The heating element will be heated until the measured temperature difference between the first temperature sensor and the second temperature sensor will become very small and substantially equal to zero.

When the temperature difference measured between the first and the second temperature sensor is zero, the heat flux inside the temperature sensing device will be zero as well. If there is no heat flux through the temperature sensing device, the heat flux from the body to the temperature sensing device will also be close to zero. If there is no heat flux between the body and the temperature sensing device, it may be assumed that the temperature sensing device has the same temperature as the core of the body.

The need for a continuous warming element in an active sensor makes this technology less favorable for implementation in wearables for reasons of energy consumption.

Passive sensors on the other hand do not require a heating element and use heat flows through the sensor to estimate CBT. These sensors include single and dual heat flux methods. Single heat flux methods are based on measuring a single heat flow through a well-insulated sensor, whereas dual heat flux methods two use heat flows through materials of different thermal resistance or thickness to derive the core body temperature. Although both technologies have shown promising results, ergonomics and underlying skin anatomy leading to uneven thermal distribution still remain an issue, with most of the previous approaches proposing a forehead patch, or even two such patches.

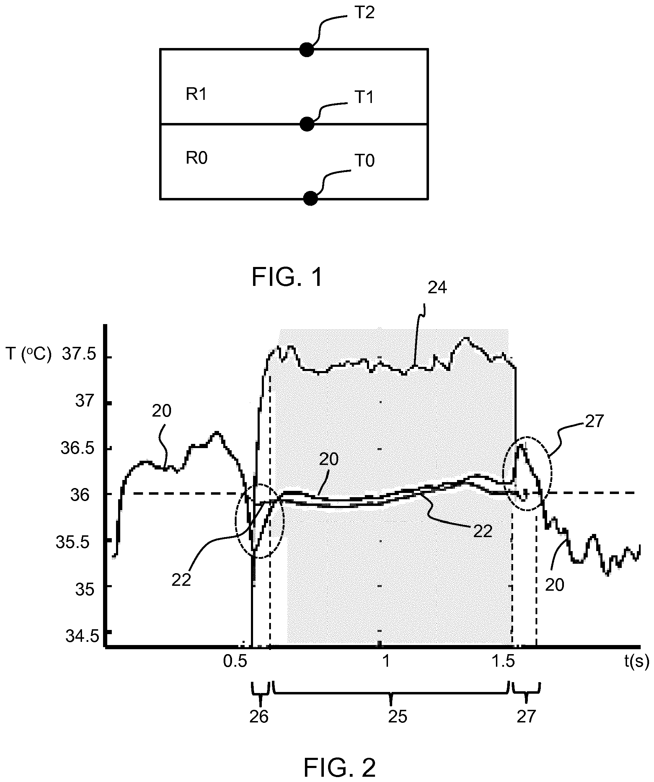

Figure 1 shows a simplified heat flow representation between two layers and in which a pair of temperature sensors is used to measure the flow across one of the layers. The lower layer with thermal resistance R0 represents the tissue between the core body area and the skin, while the upper layer with resistance R1 represents a thermally insulating material on top of the skin. The temperature sensors are placed in two locations: one sensor near the skin, measuring T1 (the bottom or skin temperature) and a second sensor on top of the insulating material, measuring T2, the top temperature.

The heat flow starting from the core body temperature T0, which is the temperature to be estimated, outwards can be modeled based on a linear flow as:

$$T0 = T1 + R0(T1-T2)/R1$$

Equivalently:

$$T0 = T1 + \alpha \cdot (T1-T2)$$

Where $\alpha$ = R0/R1.

R0 is the thermal resistance of the body (area under the sensor).

R1 is the thermal resistance of the sensor measured between the bottom layer and the top (insulated) layer.

This sensor can provide a means to estimate the core body temperature unobtrusively. For correct operation however, it needs a rather well defined value of R0. This parameter is basically a combination of tissue properties, which is specimen-dependent but varies also with location, and the thermal contact between sensor and skin surface.

The equation above is also valid for the stationary case where the thermal flow is in equilibrium, which is clearly not the case for the sensor during heating up and cooling down stages.

There are various problems with this passive sensor approach.

A first problem is that the temperature measurement may lose accuracy when rapid changes in the surrounding/environment temperature occur. A second problem is during sensor application, a desired sensor adhesion to the skin may not be achieved, and the adhesion may deteriorate over time. A mechanism for detecting this condition would be desirable.

US 2007/0282218 discloses a core body temperature monitoring system in which temperatures and rates of change of temperature are used in the calculations.

SUMMARY OF THE INVENTION

The invention is defined by the claims.

According to examples in accordance with an aspect of the invention, there is provided a core body temperature sensor system comprising:

> a first set of at least three temperature sensors for placement against the skin of a subject for whom the core body temperature is to be measured;
> a first insulating layer over the first set of temperature sensors;
> a second set of at least three temperature sensors, each temperature sensor of the second set being positioned directly over an associated temperature sensor of the first set to form a temperature sensor pair, spaced by an associated portion of the first insulating layer;
> a processor for processing the signals from the temperature sensors to derive a core body temperature,
> wherein the processor is adapted to:

>> monitor the sensed temperatures at the temperature sensors;
>> monitor a rate of change of sensed temperature at one or more of the temperature sensors;
>> determine the core body temperature based on a first representative temperature for the first set of temperature sensors, a second representative temperature for the second set of temperature sensors and rate of change information.

This sensor has multiple sensor pairs (at least three), and each pair may be used to monitor heat flow. Representative bottom and top temperatures are obtained, and this may for example comprise selection of the best sensor pair. In addition to monitoring temperatures, rate of change information is monitored. This makes the core body temperature determination more accurate, and in particular it provides a way to take account of ambient temperature fluctuations (for example as caused by a Bair Hugger) without actually monitoring the ambient temperature directly.

The processor may be adapted to determine a quality parameter (for example based on detecting large fluctuations in surrounding temperature) and generate a message in response to low quality. This message may be used to indicate that the core body temperature readings may be inaccurate at that time. It may be provided as an output or it may be appended to the core body temperature data. However, by taking account of temperature derivatives, the core body temperature determination compensates for such variations. Thus, there may only be short time periods when the core body temperature determination may be indicated as being potentially unreliable.

The processor may be adapted to determine that the system is not correctly applied to a subject and generate an output alarm. This provides a way to advise a user that the sensor system should be reapplied.

The processor is for example adapted to determine that the system is not correctly applied if a difference in sensed temperatures between the temperature sensors of the first set exceeds a threshold. If there are large temperature differences sensed by the first set, which should all be in contact with the skin, this indicates that a sensor has become spaced from the skin.

The first representative temperature is for example a mean or maximum sensed temperature for the first set. In the case of a maximum sensed temperature, this corresponds to selection of one sensor.

The second representative temperature may also be a mean or maximum sensed temperature for the second set. Alternatively it may be the temperature sensed by the temperature sensor paired with a sensor of the first set which provided the first representative temperature. In this latter case, this corresponds to the selection of a best sensor pair for use in the core body temperature determination.

The system may comprise exactly three sensor pairs arranged in locations which form an isosceles triangle. This forms a generally Y-shaped arrangement.

The first insulating layer preferably extends laterally all around the location of each sensor and between each sensor. In this way, the temperature sensor signals are minimally affected by lateral heat transfer between the different sensor locations.

For example, the first insulating layer may have shape of a central hub at which one sensor is located, a set of spokes for the other sensors and a spoke for an electrical interconnection. For a three sensor pair system, this defines a Y-shape, with one limb as the interconnect, two sensor limbs and a sensor at the middle junction.

The system preferably comprises a second insulating layer over the first insulating layer. The heat transfer characteristics of the two layers are taken into account in the determination of the core body temperature.

The second insulating layer preferably has the same shape as the first insulating layer.

The core body temperature is for example determined as:

$$T_c = T_b + \alpha(T_b - T_t) + \beta\frac{dT_t}{dt} - \gamma\frac{dT_b}{dt}$$

wherein $T_b$ is the first representative temperature, $T_t$ is the second representative temperature, $T_c$ is the core temperature and $\beta$ and $\gamma$ are calibration constants. The calibration constants depend on the thermal conductivities and specific heat capacities of the various components in the system.

The invention also provides a core body temperature measuring method comprising:

obtaining temperature measurements from a first set of at least three temperature sensors placed against the skin of a subject;

obtaining temperature measurements from a second set of at least three temperature sensors, each temperature sensor of the second set being positioned directly over an associated temperature sensor of the first set to form a temperature sensor pair, spaced by an associated portion of an insulating layer;

processing the signals from the temperature sensors to derive a core body temperature by:

monitoring the sensed temperatures at the temperature sensors;
monitoring a rate of change of sensed temperature at one or more of the temperature sensors; and
determining the core body temperature based on a first representative temperature for the first set of temperature sensors, a second representative temperature for the second set of temperature sensors and rate of change information.

The method may comprise determining that the system is not correctly applied to a subject and generating an output alarm. The method may comprise determining that the system is not correctly applied if a difference in sensed temperatures between the temperature sensors of the first set exceeds a threshold.

The method may also comprise determining a quality parameter for the core body temperature determination and generating a message in response to quality below a threshold. This may be used to indicate when it is known that the core body temperature estimation is not accurate.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a simplified heat flow representation between two layers and in which a pair of temperature sensors is used to measure the flow across one of the layers;
Figure 2 shows the calculated core temperature compared to a reference esophageal temperature measurement over the duration of a surgical procedure;
Figure 3 shows an example of the temperature sensor of the invention in cross section;
Figure 4 shows in plan view the layout of the three pairs of temperature sensors;
Figure 5 shows the temperature sensor system over the skin and two electrical models;
Figure 6 shows the result of simulation as plots of voltage (representing temperature) versus time;
Figure 7 shows the response for a first core body temperature estimation equation;
Figure 8 shows the response for a second core body temperature estimation equation;
Figure 9 shows a correctly attached sensor arrangement on the left and one in which the side sensors are lifted on the right;
Figure 10 is used to show how this detachment and the use of a Bair Hugger may alter the core body temperature estimate; and
Figure 11 shows a core body temperature measuring method.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.
The invention provides a core body temperature sensor system which has multiple pairs of temperature sensors across a first insulating layer. The sensed temperatures are monitored as well as the rate of change of sensed temperature at some or all of the temperature sensors. The core body temperature is obtained based on a first representative temperature for the first set of temperature sensors, a second representative temperature for the second set of temperature sensors and rate of change of temperature information.
As mentioned above, one problem with passive temperature sensing based on temperature flux measurement is to ensure accurate core temperature measurement when rapid changes in the surrounding/environment temperature occur. Normal environmental temperatures vary slowly and can be handled quite well by the sensor system, but in case the temperature of the patient is actively influenced using a thermal blanket (called a Bair Hugger), the changes are significantly faster than the response speed of the sensor system and errors in the readings occur in case no measures are taken.
The invention make use of time-derivative temperature information in order to determine the core body temperature. This additional information for example means the core body temperature determination distinguishes between four regimes of external conditions: Normal, Bair Hugger settled, Bair Hugger rising and Bair Hugger falling. Normal is the condition which is typical with the sensor exposed to room ambient temperature, 18-20 °C.
During an operation, the patient temperature is maintained using the so-called Bair Hugger, or thermal blanket. This is a cover with a supply of warm air which can raise the temperature locally to around 43 °C in the area surrounding the temperature sensor system, particularly one placed close to the ear.
Figure 2 shows the calculated core temperature as plot 20 as compared to the reference esophageal temperature measurement of plot 22 over the duration of a surgical procedure using the simple heat flow model above. Plot 24 indicates the measured surrounding temperature in the vicinity of the core temperature sensor.
It is observed that the calculated temperature 20 and the reference 22 agree well in the Bair Hugger settled range 25. However, the calculated temperature is typically significantly underestimated during the period 26 of raising surrounding temperature (Bair Hugger rising) and overestimated during the period 27 of decreasing surrounding temperature (Bair Hugger falling). Clearly, the standard heat flow model fails to provide an accurate temperature estimation during rapid changing environmental temperatures.

As also mentioned above, a second problem is related to quality measurement feedback during the sensor application and during the measurement, when deterioration of the sensor adhesion to the skin may occur.

There are thus two conditions in which a conventional sensor and passive heat flow model fail to provide an accurate core temperature estimation. One is poor adhesion, leading to suboptimal thermal coupling of the sensor to the skin and adherence on a curved 3-dimensional body shape. The other is related to temperature transient regimes caused either by the sensor response time when connected to or disconnected from the skin, or by rapid changes of the ambient temperature with respect to the sensor response time, such as applying the thermal blanket during a procedure as explained above.

The invention provides an improved sensor system which takes account for these rapid changes and/or enables a warning to be provided to the user if the quality of the adhesion or of the measurement is not good.

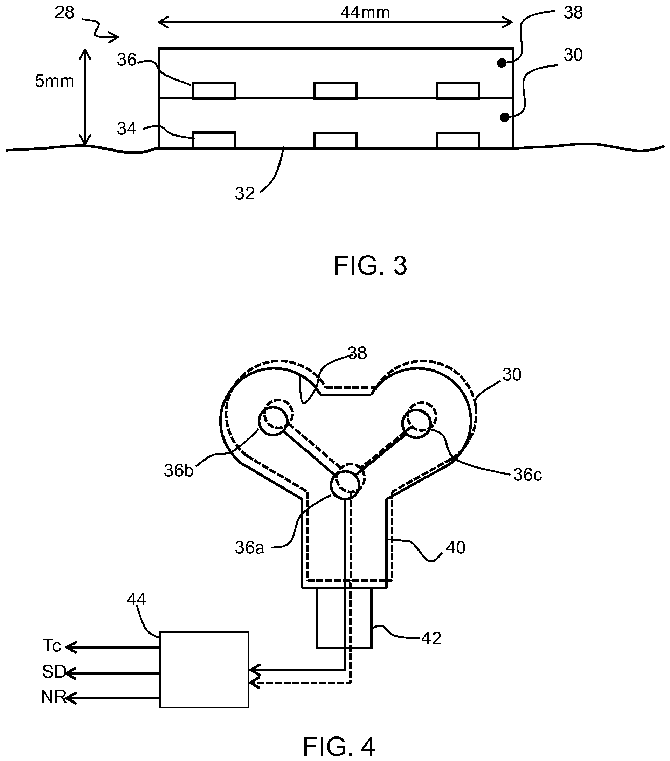

Figure 3 shows an example of the temperature sensor system of the invention in cross section. It comprises a lower insulating layer 30 which defines a skin contact surface 32 at which there is a first set 34 of three temperature sensors. A second set 36 of vertically aligned temperature sensors is provided at the top of the lower insulating layer 30. The temperature sensors thus formed vertically aligned pairs. A top insulating layer 38 covers those sensors. The individual sensor signals are provided to a processor 44 which performs the signal analysis explained below. By way of example, a sampling rate of around 1Hz may be used, i.e. signals are captured each second.

The processor outputs a core body temperature Tc but optionally also a warning SD of sensor detachment and optionally also a warning NR that the core body temperature is not reliable at a particular time.

Figure 4 shows in plan view the layout of the three pairs of temperature sensors. The top insulating layer 38 and temperature sensors 36 are shown with solid lines and the bottom insulating layer 30 and temperature sensors 34 are shown dotted.

The sensors in each layer are arranged as central sensor e.g. 36a and two limb sensors e.g. 36b, 36c with one at each side of the central sensor. The three sensors thus form an isosceles triangle. This forms a generally Y-shaped arrangement for the two insulating layers.

The two insulating layers extend laterally all around the location of each sensor and between each sensor. In this way, the temperature sensor signals are minimally affected by lateral heat transfer between the different sensor locations. For example, the overall width of the sensor system may be 44mm as shown in Figure 3 with a depth of 5mm. There is an area around each sensor of insulating material of at least twice the vertical spacing between the sensor pairs. For example, the vertical gap between sensor pairs may be 1-2mm and the sensors are laterally surrounded by at least 2mm, for example at least 3mm, of insulating material.

The top insulating layer 38 also helps to reduce or eliminate lateral heat flow between the measurement positions, which simplifies the sensor design and improves its accuracy.

The insulating layers thus have a shape of a central hub at which one sensor is located e.g. 36a, a set of spokes for the other sensors e.g. 36b, 36c and a spoke 40 for an electrical interconnection 42.

The insulating layers are able to deform by bending about the central axis (which is parallel to the spoke 40) so that it may deform to match the shape of the underlying skin surface. It may also deform about a perpendicular axis. Each spoke functions as a mechanically decoupled leaf, each with its own pair of temperature sensors. This allows the selection of the pair of sensors which operates under the best conditions, in particular the best combination of adhesion and also the response of the underlying tissue structure.

The three skin side temperature sensors 34 measure temperatures T1, T2 and T3 and the three top temperature sensors 36 measure temperatures T4, T5 and T6 respectively, so that T1 and T4 form a pair, T2 and T5 form a pair and T3 and T6 form a pair.

The selection of which pair of temperature sensors is most suitable for providing the core body temperature estimation may be performed by an algorithm which monitors the signal variation over time provided by each pair of temperature sensors.

The following values are defined:

$Tb$ = bottom temperature
$Tt$ = top temperature
$Tc$ = body core temperature
$Ts$ = ambient surrounding temperature
$\alpha$ = R0/R1, where R0 is the thermal resistance of the body (the area under the sensor) and R1 is the thermal resistance of the sensor measured between the bottom layer and the top (insulated) layer, i.e. the spacing between the sensors of a pair.

To calculate a core body temperature, a single sensor pair may be used. The value Tb is a first representative temperature for the first set of temperature sensors, and the value Tt is a second representative temperature for the second set of temperature sensors.

Tb may be the temperature measured at one of the bottom sensors. However, more generally, Tb = f(T1, T2, T3), where f is a function such as the mean or the maximum. Selection of a maximum value is equivalent to selecting one of the temperature sensors.

Again, more generally, Tt = g(T4, T5, T6), where g is a function such as the mean or the maximum. Selection of a maximum value is again equivalent to selecting one of the temperature sensors. The value Tt may be selected as the top sensor of a pair for which the bottom sensor is providing the first representative value.

The sensor design contains foam layers in combination with the temperature sensors, and as a result the temperature sensor does not respond instantaneously to a change in the environment temperature. The foam layer acts as a resistive element, while the temperature sensor (thermistor) and its immediate surrounding acts as a heat capacitor. The combination of the two introduce a time delaying effect.

If this time delay effect is ignored (i.e. dTs/dt is zero), the simple relation holds:

$$T_c = T_b + \alpha(T_b - T_t) \tag{1}$$

However, if changes in environmental conditions are taken into account, i.e. dTs/dt is not zero, the relation becomes:

$$T_c = T_b + \alpha(T_b - T_t) + \beta\frac{dT_s}{dt}$$

where $\beta$ is a calibration constant which depends on the thermal properties of the system.

To avoid the need for direct ambient temperature sensing, the surrounding temperature Ts can be estimated based on a change in the temperature difference Tt-Tb:

Thus:

$$T_c = T_b + \alpha(T_b - T_t) + \beta\frac{d(T_t - T_b)}{dt}$$

More generally:

$$T_c = T_b + \alpha(T_b - T_t) + \beta\frac{dT_t}{dt} - \gamma\frac{dT_b}{dt} \tag{2}$$

This provides a dynamic model in which the core body temperature is determined based on a first representative temperature Tb for the first set of temperature sensors, a second representative temperature Tt for the second set of temperature sensors and rate of change information.

This rate of change information relates to the two representative temperatures or a difference between them.

The effectiveness of this approach has been modeled using an electronics simulation tool.

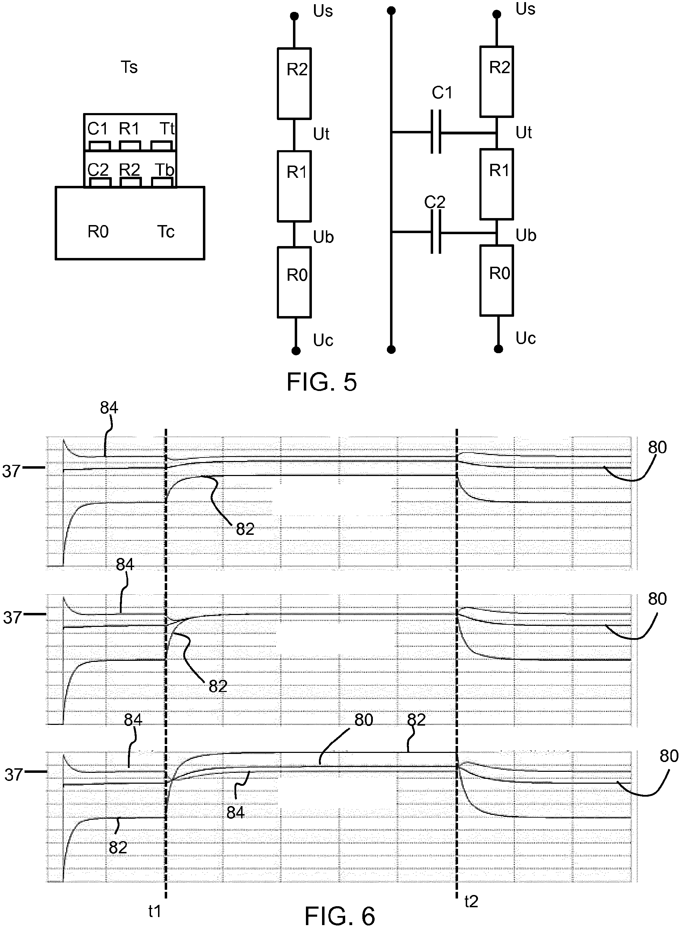

Figure 5 shows the temperature sensor system over the skin, to show the thermal resistance values R1, R2, the temperature values Tb, Tt, Tc and Ts and the thermal capacity values C1 and C2 for the two insulating layers.

A basic model is next shown in schematic form, in which the thermal capacity values C1 and C2 are not taken into account. The voltages U represent temperatures. The more complete model is shown on the right including capacitors as well as resistors.

This more complete model (of which Figure 5 still shows only a simplification) is used to form a lumped element model in the electronics simulation tool. Modeling the sensor assembly as a lumped element electronics model consisting of resistors and capacitors first involves the conversion of the thermal resistances and heat capacities into resistors and capacitors. This may be achieved by discarding lateral flow and assuming only vertical heat flow.

The model takes account of the insulating layer thicknesses (e.g. bottom layer thickness 3mm and top later thickness 2mm), the materials and their volumes of the thermistor temperature sensors and the conductive foil on which they are provided and the heat loss from the top layer to the ambient surroundings.

The element values are for example calculated for a reference size such as a 5 mm diameter section of the sensor. The 5 mm diameter is estimated as a good approximation in combination with the sizes of the thermistors.

The complete model for example includes representation using electrical circuit elements of the deep tissue of the body, the fat tissue, the skin tissue, the bottom thermistor temperature sensor, the lower insulating layer, the top thermistor temperature sensor and the top insulating layer.

The core body temperature and ambient temperature are represented in the model as voltage sources representing 37 degrees for body temperature and 20 degrees for ambient temperature. A pulse voltage source may be added on top of the ambient source to simulate the introduction of a Bair Hugger.

Switches may be used to simulate the contacting of the sensor to the body by simultaneously opening a contact to the environment temperature and closing a contact to the skin surface temperature. Capacitors are used represent the heat capacity of the thermistors, including the used solder and part of the flex foil with copper tracks.

Figure 6 shows the result of the simulation as plots of voltage (representing temperature) versus time. There are three sets of plots, the top plot is for adding a Bair Hugger with 10 degrees additional temperature, the middle plot is for adding a Bair Hugger with 17 degrees additional temperature and the bottom plot is for adding a Bair Hugger with 27 degrees additional temperature.

The plots show the bottom thermistor temperature as plot 80, the top thermistor temperature as plot 82 and the calculated core temperature as plot 84 using the basic model of equation (1). The Bair Hugger is applied at time t1 and removed at time t2. The grid width is 400 seconds so the time interval between t1 and t2 is 2000 seconds.

Figure 6 shows that there is initially (before time t1) an over-estimation of the core temperature, i.e. plot 84 is above 37 degrees, due to the dynamic behavior. Also the drop after applying the Bair Hugger and the rise after applying the Bair Hugger are present. The simulation tool was used to calculate time-derivatives of the node voltages and to combine these to form new signals.

The duration of the undershoot (immediately after time t1) and the overshoot (immediately after time t2) increases as the Bair Hugger temperature increases. These periods of undershoot and overshoot correspond to times when the core temperature calculation cannot be relied upon.

Two versions of dynamic model have been configured, which improve the dynamic behavior of the calculated core temperature considerably.

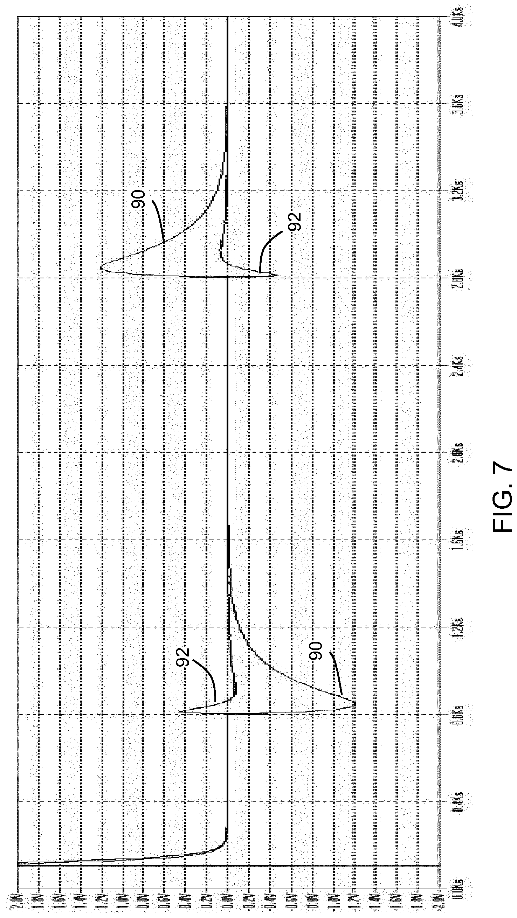

Figure 7 shows the response for:

$$Tc = Tb+0.34\cdot(Tb\text{-}Tt)+135\cdot(dTb/dt)\text{-}2\cdot(dTt/dt) \qquad (3)$$

Plot 90 is the error between the estimated core temperature using the model of Figure 6 where $Tc = Tb+0.34\cdot(Tb\text{-}Tt)$ and the actual value of 37 degrees, and plot 92 is the error between the estimated core temperature using the model of Equation (3) and the actual value of 37 degrees.

This model reduces the maximum error by almost a factor three, but more importantly, the total time period in which the result does not meet the requirement (error > $\pm$ 0.2 K) reduces by approximately a factor of eight.

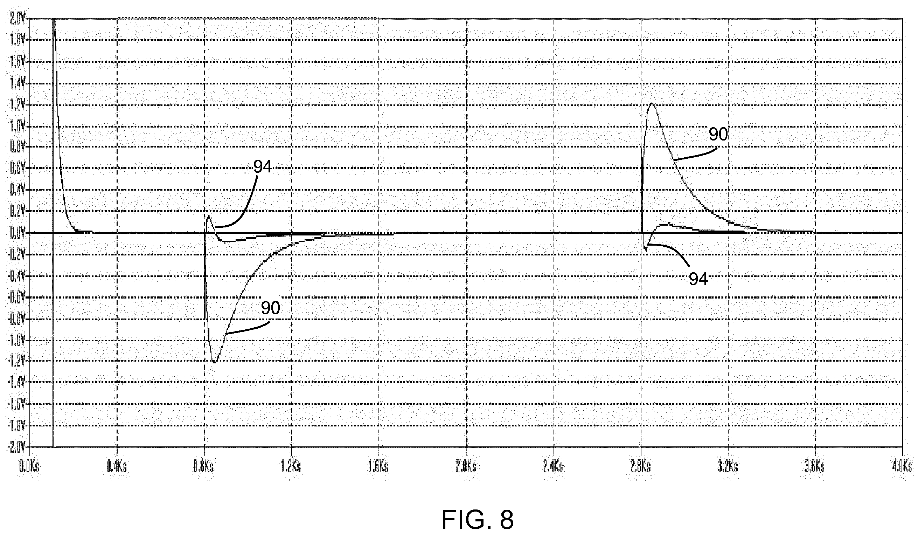

Figure 8 shows the response for:

$$Tc = Tb+0.34\cdot(Tb\text{-}Tt)+95\cdot(dTb/dt)+4\cdot(d(10\cdot Tb\text{-}Tt)/dt) \qquad (4)$$

Plot 90 is again the error between the estimated core temperature using the model of Figure 6 where $Tc = Tb+0.34\cdot(Tb\text{-}Tt)$ and the actual value of 37 degrees, and plot 94 is the error between the estimated core temperature using the model of Equation (4) and the actual value of 37 degrees.

With this model, a further reduction of the error on the Tc estimation during ambient variations is realized. Only during a short period the error raises above the allowed limit of 0.2 K.

The models above are examples only and are based on a simple 1-dimensional representation of the more complex 3-dimensional structure of the human body. Thus, more complex models may be used for better simulations.

However, even the two models above, when applied to data from clinical trials show significant improvement, indicating a decrease of the deviations in the Bair Hugger raising and Bair Hugger falling periods.

Another aspect of the system described above is to provide an indicator of adhesion quality. Sometimes the quality of the sensor adhesion is visible by the nurse (e.g. seeing a small detached area of the sensor), but in some situations it is difficult to assess.

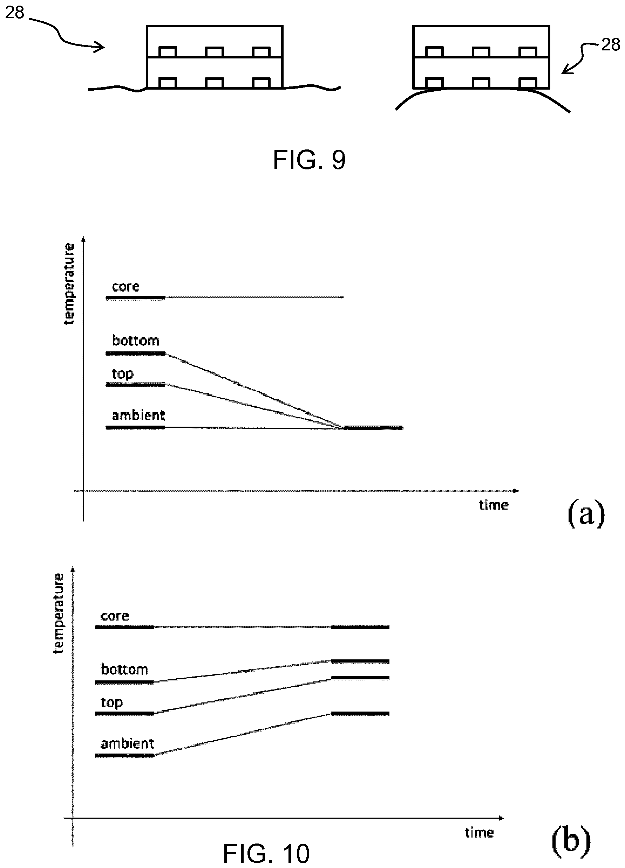

Figure 9 shows a correctly attached sensor arrangement 28 on the left and one in which the side sensors are lifted on the right. For the edge sensors, there is an air gap so that the bottom sensors are not thermally coupled directly to the skin.

This condition can be detected based on a difference in sensed temperatures between the temperature sensors of the first (bottom) set which exceeds a threshold. Thus if max{|T1-T2|,|T2-T3|,|T1-T3}> Threshold, this indicates that the sensor is not attached properly. Otherwise the attachment is optimal. The threshold is for example 0.05 degrees Celsius. Poor attachment may be used to trigger an alarm to tell the user or nurse to reapply the sensor patch.

A more complete error measure for determining adhesion quality may be defined as:

$$0.5 * smooth \left( \sqrt{\max\{|T_{b1} - T_{b2}|, |T_{b2} - T_{b3}|, |T_{b3} - T_{b1}|\} * \max\{|T_{t1} - T_{t2}|, |T_{t2} - T_{t3}|, |T_{t3} - T_{t1}|\}}, 119 \right)$$

The smoothing function calculates running root mean square value over a series of frames, as defined by the last term (119). In this case, this corresponds to a running time window of approximately 120 seconds. Note that this the default MATLAB smoothing function with a filter aperture of 119.

The maximum is chosen from the bottom temperature differences, and the maximum is chosen from the top differences, resulting in one multiplication for each time sample. This results in a time series, to which the smoothing function is applied. The smoothing function may be a moving average or a Gaussian filter.

A low error measure, below 0.1, corresponds to low measurement error. A high value indicates the sensor was not well attached.

This detachment condition may be monitored continuously and not only during initial application of the sensor system.

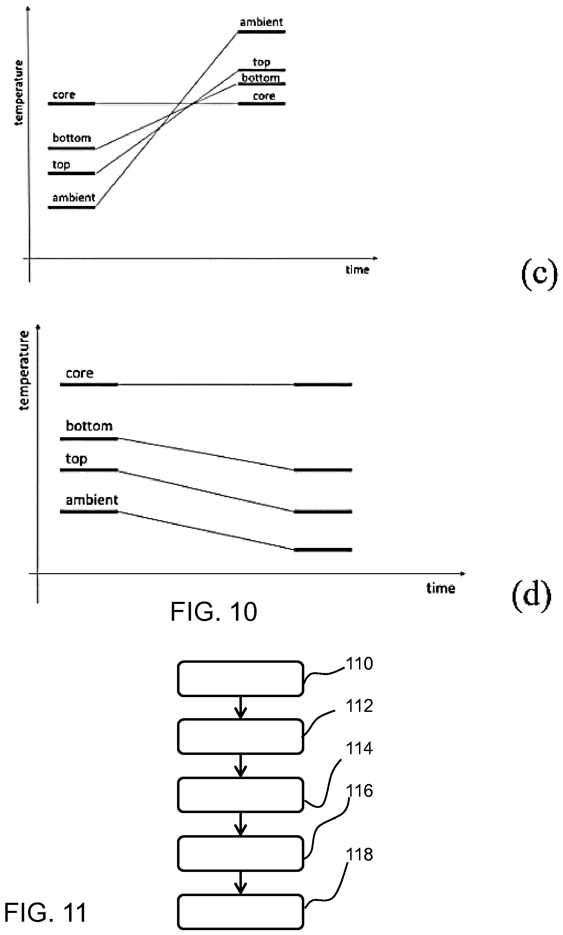

Figure 10 is used to show how this detachment and the use of a Bair Hugger may alter the core body temperature estimate.

Figure 10(a) shows what happens during sensor detachment. The top and bottom sensors of the detached sensor pair approach the ambient temperature (since this is then present on both sides of the sensor system.

Figure 10(b) shows what happens during a sudden raise in ambient temperature but with the ambient still below body temperature. The ambient temperature increases and accordingly the top and bottom sensor readings increase.

Figure 10(c) shows what happens during a sudden raise in ambient temperature to a level higher than body temperature, i.e. after application of a Bair Hugger. The ambient temperature increases beyond the core, and the top and bottom sensors show inverted readings, i.e. the top sensor temperature is higher than the bottom sensor temperature.

Figure 10(d) shows what happens during a sudden fall in temperature.

It is clear that it is possible to define a measure which looks at the change in slope of the top and bottom temperature sensor readings.

A quality parameter may be used as a measure of the degree of transient change.

This quality parameter may be defined as:

$$10 * smooth \left( \sqrt{\max\left\{\frac{dT_{b1}}{dt}, \frac{dT_{b2}}{dt}, \frac{dT_{b3}}{dt}\right\} * \max\left\{\frac{dT_{t1}}{dt}, \frac{dT_{t2}}{dt}, \frac{dT_{t3}}{dt}\right\}}, 119 \right)$$

The smoothing function again calculates the running root mean square value over a series of frames, as defined by the last term (119). In this case, this corresponds to a running time window of approximately 120 seconds. Note that this the default MATLAB smoothing function with a filter aperture of 119.

The maximum is chosen from the bottom temperature gradients, and the maximum is chosen from the top temperature gradients, resulting in one multiplication for each time sample. This results in a time series, to which the smoothing function is applied. The smoothing function may again be a moving average or a Gaussian filter.

Faster measures can be constructed using only one pair of thermistors or a smaller window of smoothing, without compromising on the accuracy of the quality parameter.

The quality parameter may instead be based on thresholding the temperature derivatives. Suitable thresholds can be derived as a result of training algorithm applied to a sample of data for which the performance is known.

When this quality parameter is large, the bottom sensors behave differently to the top sensors. This parameter may be used an error indicator, and it has been shown that it follows the same trend as the absolute value of the error value , i.e. plots 92 and 94 in Figures 7 and 8.

This quality parameter may be compared with a threshold. If the threshold is exceeded so that the quality is below a corresponding threshold, it is determined that the core body temperature is not to be relied upon during that time. Thus, a message may be provided for this purpose. Any actions such as an alarm which may be triggered by particular core body temperature values may be inhibited during the times when the core body temperature estimation is not to be relied upon. The quality indication may be attached to recorded data as a flag. However, the approach of the invention, making use of the temperature derivatives, makes these time periods very short.

The invention also provides a core body temperature measuring method comprising:

in step 110, obtaining temperature measurements from a first set of at least three temperature sensors placed against the skin of a subject;

in step 112 obtaining temperature measurement from a second set of at least three temperature sensors, each temperature sensor of the second set being positioned directly over an associated temperature sensor of the first set to form a temperature sensor pair, spaced by an associated portion of (or being in a direct contact with) an insulating layer; and

in step 114 processing the signals from the temperature sensors to derive a core body temperature. This involves monitoring the sensed temperatures at the temperature sensors as well as rates of change of sensed temperature.

In step 116 it is determined that the system is not correctly applied to a subject and an output alarm is generated.

In step 118 it is determined that a quality has dropped below a threshold (based on determination of a quality parameter) so that the estimated core body temperature is not to be relied upon and this information is provided as an output or as additional information (metadata).

The invention is for example of interest for monitoring of the core temperature of neonates, significantly reducing interventions to the neonates.

As discussed above, the system makes use of processor to perform the data processing. The processor can be implemented in numerous ways, with software and/or hardware, to perform the various functions required. The processor typically employs one or more microprocessors that maybe programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A core body temperature sensor system comprising:

a first set (34) of at least three temperature sensors for placement against the skin of a subject for whom the core body temperature is to be measured;

a first insulating layer (30) over the first set of temperature sensors;

a second set (36) of at least three temperature sensors, each temperature sensor of the second set being positioned directly over an associated temperature sensor of the first set to form a temperature sensor pair, spaced by an associated portion of the first insulating layer;

a processor (44) for processing the signals from the temperature sensors to derive a core body temperature, wherein the processor is adapted to:

monitor the sensed temperatures at the temperature sensors;

monitor a rate of change of sensed temperature at one or more of the temperature sensors;

determine the core body temperature based on a first representative temperature for the first set of temperature sensors, a second representative temperature for the second set of temperature sensors and rate of change information.

**2.** A system as claimed in claim 1, wherein the processor is adapted to determine a quality parameter for the core body temperature determination and generate a message (NR) in response to quality below a threshold.

**3.** A system as claimed in claim 1 or 2, wherein the processor (44) is adapted to determine that the system is not correctly applied to a subject and generate an output alarm.

**4.** A system as claimed in claim 3, wherein the processor is adapted to determine that the system is not correctly applied if a difference in sensed temperatures between the temperature sensors of the first set exceeds a threshold.

**5.** A system as claimed in any one of claims 1 to 4, wherein the first representative temperature is a mean or maximum sensed temperature for the first set.

**6.** A system as claimed in claim 5, wherein the second representative temperature is a mean or maximum sensed temperature for the second set, or the temperature sensed by the temperature sensor paired with a sensor of the first set which provided the first representative temperature.

**7.** A system as claimed in any one of claims 1 to 6 comprising exactly three sensor pairs arranged in locations which form an isosceles triangle.

**8.** A system as claimed in any one of claims 1 to 7 wherein the first insulating layer (30) extends laterally all around the location of each sensor and between each sensor.

**9.** A system as claimed in claim 8, wherein the first insulating layer has shape of a central hub at which one sensor is located, a set of spokes for the other sensors and a spoke for an electrical interconnection.

**10.** A system as claimed in any one of claims 1 to 9, comprising a second insulating layer (38) over the first insulating layer.

**11.** A system as claimed in claim 10, wherein the second insulating layer has the same shape as the first insulating layer.

**12.** A system as claimed in any one of claims 1 to 11, wherein the core body temperature is determined as:

$$T_c = T_b + \alpha(T_b - T_t) + \beta \frac{dT_t}{dt} - \gamma \frac{dT_b}{dt}$$

wherein Tb is the first representative temperature, Tt is the second representative temperature, Tc is the core temperature and $\beta$ and $\gamma$ are calibration constants.

**13.** A core body temperature measuring method comprising:

(110) obtaining temperature measurements from a first set of at least three temperature sensors placed against the skin of a subject;
(112) obtaining temperature measurement from a second set of at least three temperature sensors, each temperature sensor of the second set being positioned directly over an associated temperature sensor of the first set to form a temperature sensor pair, spaced by an associated portion of an insulating layer; and
(114) processing the signals from the temperature sensors to derive a core body temperature by:

monitoring the sensed temperatures at the temperature sensors;
monitoring a rate of change of sensed temperature at one or more of the temperature sensors; and
determining the core body temperature based on a first representative temperature for the first set of temperature sensors, a second representative temperature for the second set of temperature sensors and rate of change information.

**14.** A method as claimed in claim 13, comprising determining a quality parameter for the core body temperature determination and generating a message (NR) in response to quality below a threshold.

**15.** A method as claimed in claim 13 or 14, comprising (116) determining that the system is not correctly applied to a subject and generating an output alarm, for example if a difference in sensed temperatures between the temperature sensors of the first set exceeds a threshold.

FIG. 1

FIG. 2

28

44mm  38

5mm  36  30

34

32

## FIG. 3

38

30

36b

36c

36a

40

42

44

Tc

SD

NR

## FIG. 4

FIG. 5

FIG. 6

FIG. 7

FIG. 8

FIG. 9

(a)

FIG. 10

(b)

(c)

FIG. 10

(d)

FIG. 11

Europäisches
Patentamt

European
Patent Office

Office européen
des brevets

# EUROPEAN SEARCH REPORT

**Application Number**

EP 18 21 1550

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/213559 A1 (POLLACK RICHARD S [US] ET AL) 1 September 2011 (2011-09-01) * paragraphs [0030], [0035]; figures 1A,2 * | 1-11, 13-15 | INV. A61B5/01 G01K1/20 G01K13/00 |
| X | EP 1 768 546 A1 (ADVANCED MONITORS CORP [US]) 4 April 2007 (2007-04-04) * equation 3; paragraph [0043]; figures 4,7 * | 1-11, 13-15 | |
| X | WO 98/50766 A1 (MEDISIM LTD [IL]; YARDEN MOSHE [IL] ET AL.) 12 November 1998 (1998-11-12) * page 3, line 1 - page 4, line 9; figures 1,3 * | 1-11, 13-15 | |
| X | US 2007/206655 A1 (HASLETT JAMES W [CA] ET AL) 6 September 2007 (2007-09-06) * paragraph [0018]; figure 5 * | 1-11, 13-15 | |
| X | WO 2017/108964 A1 (KONINKLIJKE PHILIPS NV [NL]) 29 June 2017 (2017-06-29) * page 7, paragraph 2; figure 11 * * page 13, paragraph 3 * | 1-11, 13-15 | TECHNICAL FIELDS SEARCHED (IPC) A61B G01W G01K |
| A | US 2009/105605 A1 (ABREU MARCIO MARC [US]) 23 April 2009 (2009-04-23) * paragraph [0929] * | 1-11, 13-15 | |

The present search report has been drawn up for all claims

| Place of search | Date of completion of the search | Examiner |
|---|---|---|
| Berlin | 11 June 2019 | Clevorn, Jens |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

           

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 21 1550

This annex lists the patent family members relating to the patent documents cited in the above-mentioned European search report.
The members are as contained in the European Patent Office EDP file on
The European Patent Office is in no way liable for these particulars which are merely given for the purpose of information.

11-06-2019

| Patent document cited in search report | | Publication date | Patent family member(s) | | Publication date |
|---|---|---|---|---|---|
| US 2011213559 | A1 | 01-09-2011 | US | 2011213559 A1 | 01-09-2011 |
| | | | US | 2015297146 A1 | 22-10-2015 |
| EP 1768546 | A1 | 04-04-2007 | CN | 101031233 A | 05-09-2007 |
| | | | EP | 1768546 A1 | 04-04-2007 |
| | | | JP | 4824020 B2 | 24-11-2011 |
| | | | JP | 2008502903 A | 31-01-2008 |
| | | | US | 2005043631 A1 | 24-02-2005 |
| | | | US | 2007100564 A1 | 03-05-2007 |
| | | | WO | 2006009585 A1 | 26-01-2006 |
| WO 9850766 | A1 | 12-11-1998 | AT | 208036 T | 15-11-2001 |
| | | | AU | 724571 B2 | 28-09-2000 |
| | | | BR | 9809322 A | 04-07-2000 |
| | | | CA | 2288202 A1 | 12-11-1998 |
| | | | CN | 1257577 A | 21-06-2000 |
| | | | DE | 69802278 T2 | 18-07-2002 |
| | | | EP | 0979394 A1 | 16-02-2000 |
| | | | ES | 2165675 T3 | 16-03-2002 |
| | | | IL | 120758 A | 29-02-2000 |
| | | | JP | 3863192 B2 | 27-12-2006 |
| | | | JP | 3935915 B2 | 27-06-2007 |
| | | | JP | 2001522466 A | 13-11-2001 |
| | | | JP | 2005321412 A | 17-11-2005 |
| | | | KR | 20010012163 A | 15-02-2001 |
| | | | TR | 199902710 T2 | 21-03-2000 |
| | | | US | 6280397 B1 | 28-08-2001 |
| | | | WO | 9850766 A1 | 12-11-1998 |
| US 2007206655 | A1 | 06-09-2007 | CA | 2538940 A1 | 22-06-2006 |
| | | | US | 2007206655 A1 | 06-09-2007 |
| WO 2017108964 | A1 | 29-06-2017 | BR | 112018012423 A2 | 18-12-2018 |
| | | | CN | 108431564 A | 21-08-2018 |
| | | | EP | 3394580 A1 | 31-10-2018 |
| | | | JP | 2019501389 A | 17-01-2019 |
| | | | US | 2018364109 A1 | 20-12-2018 |
| | | | WO | 2017108964 A1 | 29-06-2017 |
| US 2009105605 | A1 | 23-04-2009 | US | 2009105605 A1 | 23-04-2009 |
| | | | US | 2013124039 A1 | 16-05-2013 |
| | | | US | 2015150453 A1 | 04-06-2015 |
| | | | US | 2017065183 A1 | 09-03-2017 |

EPO FORM P0459

For more details about this annex : see Official Journal of the European Patent Office, No. 12/82

## REFERENCES CITED IN THE DESCRIPTION

**Patent documents cited in the description**

- US 20070282218 A